# EUROPEAN PATENT SPECIFICATION

(11) **EP 0 927 056 B1**
(45) Date of publication and mention of the grant of the patent: **28.04.2004**
(21) Application number: 97939978.9
(22) Date of filing: 10.09.1997
(51) Int. Cl.: A61M 5/24, A61M 5/31

(54) **SYRINGE**
SPRITZE
SERINGUE

(30) Priority: 13.09.1996 DK 99096
(43) Date of publication of application: 07.07.1999
(73) Proprietor: NOVO NORDISK A/S, 2880 Bagsvaerd (DK)
(72) Inventor: HANSEN, Steffen, DK-3400 Hiller d (DK); KLITGAARD, Peter, Christian, DK-2765 Sm run (DK)
(74) Representative: Hansen, Einar Tronier
(86) International application number: PCT/DK1997/000379
(87) International publication number: WO 1998/010812

(56) References cited:
- EP-A- 0 697 222
- US-A- 286 605

## Description

The invention relates to syringes of the kind comprising a housing, a dose setting mechanism in the housing, an injection button, and a needle receiving member, by which syringe doses of a medicine can be set by the dose setting mechanism and by operating the injection button can be pressed out through a needle mounted on the needle receiving member from an ampoule accommodated in the housing.

Such syringes are mainly developed to be used by patients, mainly diabetics, who have to frequently inject themselves with individually set doses of insulin. The syringes are often given a shape like a fountain pen so it may be carried by the patient all through the day and is always ready for an injection. The ampoule may contain medicine enough for several days, but the used needle must be removed after an injection and a new needle mounted before the next injection. This opens the need for extra needles which the patient further must bring with him. Also other kinds of equipment may be necessary so as strips by which the blood glucose concentration may be estimated and pills if the diabetic suffers from the type II diabetes which is treated partly by pills and partly by insulin injections. Consequently the syringe is mainly delivered in a small case which accommodates the syringe, a number of needles, possibly a ampoule with medicine etc. However, this already makes the patient less tree than it was intended when the pen was developed as an device integrating all the most necessary parts. Especially the need for spare needles have to be met.

From EP 697 222 it is known to fix a tube containing a number of needles to a pen by a clip having oppositely directed C-shaped portions. This solution only transforms the inconvenience of having two parts to carry into the inconvenience of having a bulky thing to wear.

It is an object of the invention to provide a syringe by which this inconveniences are avoided.

This is obtained by a syringe according to the invention.

The compartment may according to the invention has a size enabling it to accommodate at . least one needle stored In a needle magazine.

The compartment may according to the invention be a cavity in the housing covered by a lid, or it may be an inner space in a cap which is mounted on the housing or in a drawer which is slides into the housing.

In the following the invention is further described with references to the drawing wherein,
- Figure 1: shows a syringe with a compartment according to the invention, the compartment being closed by a cap,
- Figure 2: shows the syringe in figure 1 with the cap off, and
- Figure 3: shows another embodiment of a syringe with a compartment covered by a lid.

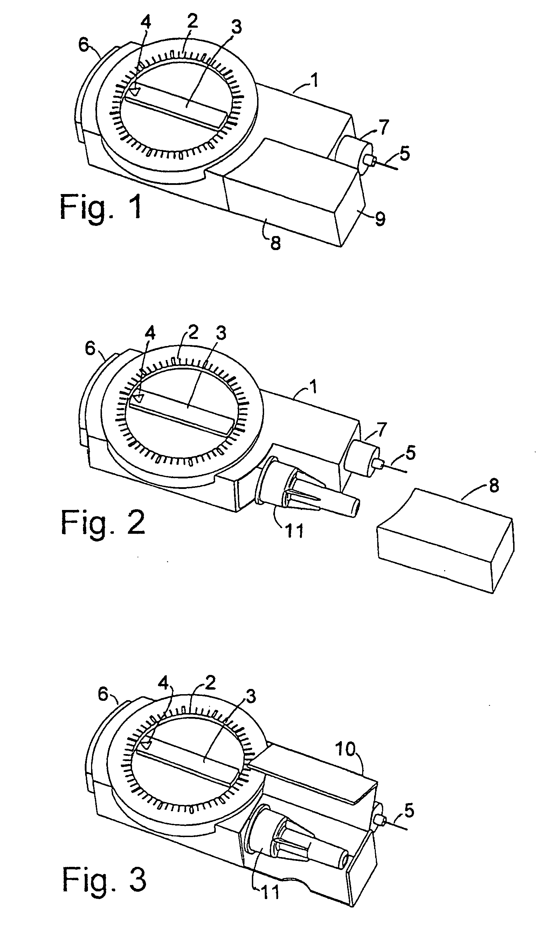

Figure 1 shows a preferred syringe having a compartment according to the invention. The syringe is of the type which due to the use of a flexible piston rod is shorter and broader than a conventional pen shaped syringe. Such syringes has a format lying between the size of a lighter and a pack of cigarettes and are consequently easy to carry in a pocket. Pen shaped syringes has through the time lost their slim design due to the fact that larger ampoules are used and the dose setting mechanism is extended by electronic displays which calls for a larger diameter of at least the proximal end of the pen. With such pen a compartment may be provided in the large diameter part of the syringe or in extensions of the pen, but such extensions will not surely make the pen more bulky. Consequently a new box shaped design as the one shown in figure 1 is preferred.

The syringe in figure 1 comprises a housing 1 carrying a scale 2 on which a dose may be set by rotating a finger grip 3 until an arrow 4 on this grip indicates the wanted dose on the scale 2. The dose may thereafter by pressing an injection button 6 be injected through a needle 5 which is carried in a needle hub 7 which is mounted on an needle receiving member on the syringe. A cap 8 covers a compartment in the housing.

Figure 2 shows the syringe of figure 1 with the cap 8 drawn off and a needle case 11 is sketched to show how such a needle case 11 which accommodates a needle as the needle 5 mounted in its needle hub 7 may be stored in the compartment. The cap is further so designed that its end surface 9 forms an abutment which may abut the skin during the injection.

Figure 3 shows a syringe corresponding to the one shown in figure 1 and 2. In this syringe the compartment is totally integral with the housing and is accessible through a lid 10.

In the drawings the equipment in the compartment is shown as a needle, but may other kinds of accessories may be stored in the compartment, e.g. electronic devices which could ordinarily be integrated in a durable syringe but which are too expensive to discard with a disposable syringe. Such electronic devices may be different types of timers or devices for electronic reading of set doses or the number of doses left in the ampoule. Also things which the patient may want to have a hand so as instructions, swaps, tablets, aids for mounting or dismounting of the needle, etc.

## Claims

1. A syringe comprising a housing (1), a dose setting mechanism (2,3,4) in the housing (1), an injection button (6), and a needle receiving member, by which syringe doses of a medicine can be set by the dose setting mechanism (2,3,4) and by operating the injection button (6) can be pressed out through a needle (5) mounted on the needle receiving member from an ampoule accommodated in the housing, **characterised in that**, the housing is box-shaped and comprises a compartment accessible for the user of such syringe for storing one or more accessories such as a needle, an electronic device, swaps, tablets, an aid for mounting or dismounting a needle or the like.

2. A syringe according to claim 1, **characterised in that**, the compartment has a size enabling it to accommodate at least one needle.

3. A syringe according to claim 1 or 2, **characterised in that**, the compartment is a cavity in the housing covered by a lid (10).

4. A syringe according to claim 1 or 2, **characterised in that**, the compartment is an inner space in a cap (8) which is mounted on the housing.

5. A syringe according to claim 1 or 2, **characterised in that**, the compartment is the inner space in a drawer which slides into the housing.

## Patentansprüche

1. Spitze, umfassend ein Gehäuse (1), einen Dosiseinstellungsmechanismus (2, 3, 4) im Gehäuse (1), einen Injektionsdruckknopf (6) und ein Nadelaufnahmeelement, durch welche Spritzendosen einer Medizin durch den Dosiseinstellungsmechanismus (2, 3, 4) eingestellt werden können und durch Bedienen des Injektionsdruckknopfes (6) durch eine am Nadelaufnahmeelement angebrachten Nadel (5), aus einer im Gehäuse untergebrachten Ampulle gedrückt werden können, **dadurch gekennzeichnet, dass** das Gehäuse schachtelförmig ist und ein für den Anwender einer solchen Spritze zugängliches Fach zur Aufbewahrung eines oder mehrerer Zubehörteile wie einer Nadel, einer elektronischen Vorrichtung, Tupfer, Tabletten, einer Hilfe zum Anbringen oder Abnehmen einer Nadel oder dergleichen umfasst.

2. Spritze nach Anspruch 1, **dadurch gekennzeichnet, dass** das Fach eine Größe aufweist, die es ermöglicht, dass mindestens eine Nadel darin untergebracht werden kann.

3. Spritze nach Anspruch 1 oder 2, **dadurch gekennzeichnet, dass** das Fach ein durch einen Deckel (10) abgedeckter Hohlraum im Gehäuse ist.

4. Spritze nach Anspruch 1 oder 2, **dadurch gekennzeichnet, dass** das Fach ein Innenraum in einer Kappe (8) ist, die am Gehäuse angebracht wird.

5. Spritze nach Anspruch 1 oder 2, **dadurch gekennzeichnet, dass** das Fach ein Innenraum in einem Schubfach ist, das in das Gehäuse gleitet.

## Revendications

1. Seringue comprenant un logement (1), un mécanisme de détermination de dose (2, 3, 4) dans le logement (1), un bouton d'injection (6), et un élément de réception d'aiguille, avec lesquels les doses de seringue d'un médicament qui peuvent être déterminées par le mécanisme de détermination de dose (2, 3, 4) et en faisant fonctionner le bouton d'injection (6), peuvent être expulsées par une aiguille montée sur l'élément de réception d'aiguille à partir d'une ampoule logée dans le logement, **caractérisée en ce que** le logement est en forme de boîtier et comprend un compartiment accessible pour l'utilisateur d'une telle seringue pour stocker un ou plusieurs accessoires tels qu'une aiguille, un dispositif électronique, des objets à échanger, des comprimés, une aide pour monter ou démonter une aiguille ou similaire.

2. Seringue selon la revendication 1, **caractérisée en ce que** le compartiment a une taille lui permettant de loger au moins une aiguille.

3. Seringue selon la revendication 1 ou 2, **caractérisée en ce que** le compartiment est une cavité située dans le logement recouverte par un couvercle (10).

4. Seringue selon la revendication 1 ou 2, **caractérisé en ce que**, le compartiment est un espace interne dans un capuchon (8) qui est monté sur le logement.

5. Seringue selon la revendication 1 ou 2, **caractérisée en ce que** le compartiment est l'espace interne dans un tiroir qui coulisse dans le logement.
